(19) Europäisches Patentamt / European Patent Office / Office européen des brevets

(11) **EP 4 449 039 B1**

(12) **EUROPEAN PATENT SPECIFICATION**

(45) Date of publication and mention
of the grant of the patent:
**18.03.2026 Bulletin 2026/12**

(21) Application number: **21840481.2**

(22) Date of filing: **15.12.2021**

(51) International Patent Classification (IPC):
***F27B 7/20*** *(2006.01)*

(52) Cooperative Patent Classification (CPC):
**C04B 7/364; C04B 7/361; F27B 7/20; F27B 7/42;**
G01N 33/383

(86) International application number:
**PCT/EP2021/085947**

(87) International publication number:
**WO 2023/110086 (22.06.2023 Gazette 2023/25)**

(54) **METHOD AND APPARATUS FOR EVALUATING RESIDUAL SULPHUR IN A CEMENT PREHEATER, METHOD FOR EVALUATING BLOCKAGE OF A CEMENT PREHEATER, AND A CEMENT PREHEATER**

VERFAHREN UND VORRICHTUNG ZUR BEWERTUNG VON RESTSCHWEFEL IN EINEM ZEMENTVORWÄRMER, VERFAHREN ZUR BEWERTUNG DER BLOCKIERUNG EINES ZEMENTVORWÄRMERS UND ZEMENTVORWÄRMER

PROCÉDÉ ET APPAREIL D'ÉVALUATION DU SOUFRE RÉSIDUEL DANS UN PRÉCHAUFFEUR DE CIMENT, PROCÉDÉ D'ÉVALUATION DE BLOCAGE D'UN PRÉCHAUFFEUR DE CIMENT ET PRÉCHAUFFEUR DE CIMENT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**23.10.2024 Bulletin 2024/43**

(73) Proprietor: **ABB SCHWEIZ AG**
**5400 Baden (CH)**

(72) Inventors:
 • **KUMAR, Subhash**
 **Bokaro, Jharkhand 828403 (IN)**
 • **MADUSKAR, Deepti**
 **Bhopal 462042 (IN)**
 • **KUMAR, Srijit**
 **Karnataka, Bangalore 560016 (IN)**

 • **SHARMA, Divyasheel**
 **Karnataka, Bangalore 560048 (IN)**
 • **R, Sandeep**
 **Karnataka, Bangalore 560091 (IN)**
 • **RAJ, Vimal**
 **Karnataka, Bangalore 560058 (IN)**
 • **PURKAYASTHA, Kallol**
 **Karnataka, Bangalore 560037 (IN)**

(74) Representative: **Zimmermann & Partner**
**Patentanwälte mbB**
**Postfach 330 920**
**80069 München (DE)**

(56) References cited:
 **WO-A1-2019/000807      JP-A- 2017 036 165**
 **US-B1- 6 383 283**

## Description

### TECHNICAL FIELD

Embodiments of the present disclosure relate to a method and a sensor device for measuring the residual sulphur in a cement cyclone, particularly a soft sensor using machine learning, for reducing or preventing blockage caused by agglomeration.

### BACKGROUND

In a cement kiln of the present state of the art, a cement preheater is used to preheat and calcinate the raw material entering the kiln using waste heat. In general, exhaust gas exiting from the cement kiln as well as raw material entering the kiln are passed through a pre-calciner and a cascading arrangement of cement cyclones, which efficiently transfers heat from the exhaust gas to the raw material, heating the raw material to temperatures of 700 °C to 900 °C and calcining the material up to 90 % prior to entering the kiln.

However, narrow passages within a cement cyclone, particularly the exits of the cement cyclone, may become blocked, causing a complete shutdown of the cement kiln to clear the blockage. A cement cyclone may become blocked by mechanical causes, for example, a mechanical failure of a duct or the presence of a foreign object, or may become blocked by chemical causes, such as agglomeration. Chemical agglomeration on the internal walls of the cement cyclone occurs when volatile compounds of the raw material and/or fuel, such as sulphur, chlorine and alkali compounds, condense and accumulate on cooler regions, leading in particular to the formation of alkali sulphates. The alkali sulphates tend to re-circulate throughout the cement preheater, and tend to accumulate on the inner walls of a cyclone duct, reducing the flow diameter of the duct and eventually causing a blockage.

In the current state of the art, determining whether a cement cyclone is blocked, or determining what percentage of a duct within a cement cyclone is obstructed, can be challenging without completely shutting down the cement kiln and inspecting the ducts internally. Japanese patent application publication JP-H11-262692A describes one method for preventing the clogging of a cement cyclone using a monitoring device having an electromagnetic wave emitter and sensor to estimate the thickness of built-up material. Based on this estimation, an air blast technique is used to remove the built-up material to alleviate the blockage. In this case, gamma radiation is emitted through a cyclone duct and detected by the sensor, and the strength of the sensed electromagnetic signal can be used to determine a level of blockage. International patent application publications WO 2013/093245 A1 and WO 2017/005697 A1 describe similar approaches to detecting a blockage using non-ionizing radiation.

However, these approaches have several disadvantages. Neither technique allows for determining whether a blockage may have resulted from a mechanical blockage or from chemical agglomeration, and the accuracy of determining a percentage of blockage is limited. Further, these methods only describe reactive approaches to blockages, where operational decisions would be made in response to a blockage. A proactive approach would be more desirable, where sufficient information on the current pathway size of the cyclone and an expected operation time until a blockage occurs is available, so that maintenance operations can be scheduled in advance. In addition, the use of some forms of radiation is harmful for maintenance personnel should they be exposed. WO 2019/000807 A1 describes determining residual sulphur in a cement preheater.

In the field of cement kilns and cement production, the use of software modelling, including machine learning and neural networks, has been increasing. Estimating and predicting the quality of cement clinker based on process variables, including the presence of sulphur, has been proposed. However, the modelling of residual sulphur recirculating in a cement cyclone, which is a primary cause of chemical agglomeration, has not yet been suggested.

In view of the deficiencies in the current state of the art, improved methods of measuring residual sulphur in a cement cyclone, and improved methods of evaluating blockages in a cement cyclone, are desired.

### SUMMARY

In view of the above challenges and problems arising in the state of the art, improved methods and apparatus for evaluating residual sulphur in a cement cyclone are sought.

According to a first aspect of the present disclosure, a method for evaluating residual sulphur in a cement preheater of a cement kiln is provided. The method includes determining values of fuel sulphur content and fuel rate of consumption of a fuel being provided to the cement kiln, determining a value of hotmeal quality of a hotmeal being provided to the cement kiln, determining a value of clinker sulphur content of a clinker being produced by the cement kiln, and evaluating the residual sulphur in the cement preheater using a sulphur evaluation unit, wherein the residual sulphur is based on the values of the fuel sulphur content, the fuel rate of consumption, the hotmeal quality and the clinker sulphur content.

According to a second aspect of the present disclosure, a method for evaluating blockage in a cement preheater is provided. The method includes evaluating the residual sulphur in the cement preheater using the method according to the first aspect, determining an agglomeration rate of sulphur compounds agglomerating on an inner surface of the cement preheater based on the residual sulphur, and evaluating a level of blockage in at least one predetermined pathway of the cement preheater using a blockage evaluation unit, wherein the level of blockage is based on the agglomeration rate.

According to a third aspect of the present disclosure, a sensor device for evaluating residual sulphur in a cement preheater of a cement kiln is provided. The sensor device includes a determining unit configured for determining values of fuel sulphur content, fuel rate of consumption, hotmeal quality and clinker sulphur content, and a sulphur evaluation unit configured for evaluating the residual sulphur using the method according to the first aspect.

According to a fourth aspect of the present disclosure, a cement preheater is provided. The cement preheater includes at least one cement cyclone, a sensor device for evaluating residual sulphur in the cement preheater according to the third aspect, and a controller configured to carry out the method for evaluating blockage in the cement preheater according to the second aspect.

According to a fifth aspect of the present disclosure, a cement kiln is provided, the cement kiln including a cement preheater according to the fourth aspect.

Aspects of the present disclosure provide methods and apparatus for evaluating an amount of residual sulphur present in the cement preheater of a cement kiln, and evaluating blockages and partial blockages of the cement preheater caused by the agglomeration of sulphur compounds. The operation and maintenance of a cement preheater having such an apparatus is improved, by allowing for efficient planning of maintenance activities, and to avoid downtime of the cement kiln caused by blockages of the cement preheater. Further, the methods and apparatus of the present disclosure allow for the rate of agglomeration to be slowed or controlled with preventative measures such as adjustment of the sulphur-alkali ratio (SAR) in the cement preheater, or to evaluate whether cleaning operations, such as an air blast operation, are required.

Those skilled in the art will recognise additional features and advantages upon reading the following detailed description, and upon viewing the accompanying drawings.

## BRIEF DESCRIPTION OF THE DRAWINGS

The components in the figures are not necessarily to scale, instead emphasis being placed upon illustrating the principles of the invention. Moreover, in the figures, like reference numerals designate corresponding parts. In the drawings:

Fig. 1        illustrates a schematic side view of a conventional cement kiln having a cement preheater;
Fig. 2        illustrates is a schematic side section view of a conventional cement cyclone;
Fig. 3a-3c   illustrates a top view of an outlet blockage in a conventional cement cyclone;
Fig. 4        is a schematic block diagram of a method for evaluating residual sulphur in a cement preheater according to embodiments of the present disclosure; and

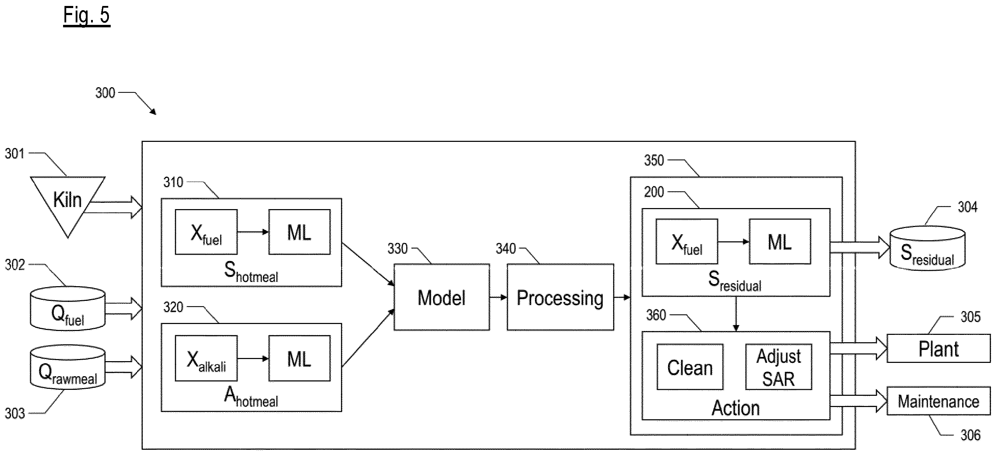

Fig. 5        is a schematic block diagram of a method for evaluating blockage in a cement preheater according to embodiments of the present disclosure.

## DETAILED DESCRIPTION

Reference will now be made in detail to the various embodiments, one or more examples of which are illustrated in each figure. Each example is provided by way of explanation and is not meant as a limitation.

Within the following description of the drawings, the same reference numbers refer to the same or to similar components. Generally, only the differences with respect to the individual embodiments are described. Unless specified otherwise, the description of a part or aspect in one embodiment applies to a corresponding part or aspect in another embodiment as well.

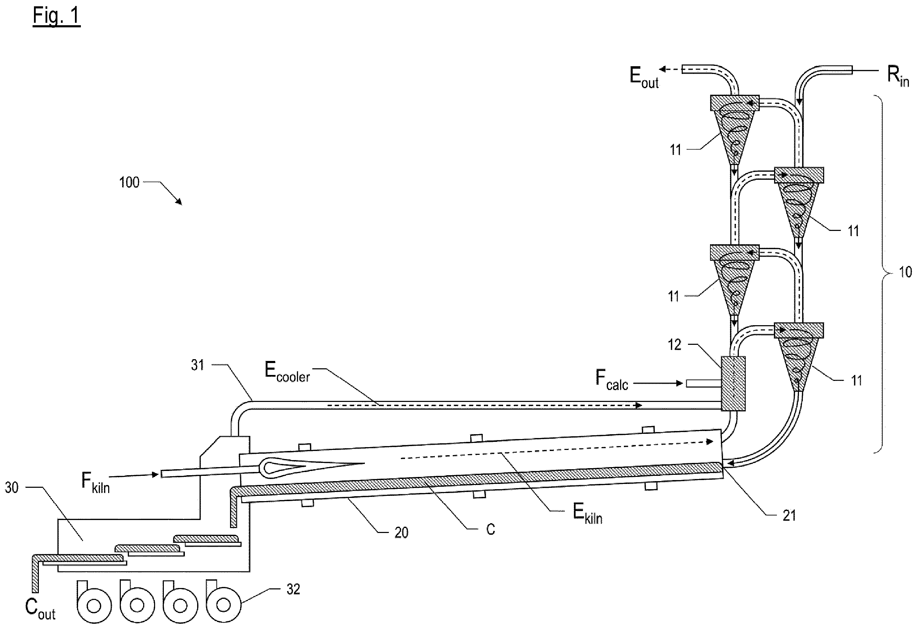

Reference will be made to Fig. 1, which shows a schematic side view of a conventional cement kiln 100 having a cement preheater 10. The cement kiln 100 includes a rotary kiln 20 having kiln entrance 21 into which hotmeal exiting from the cement preheater 10 is fed, and the rotation of the rotary kiln 20 causes it gradually to move downhill to the other end of the rotary kiln 20. At the other end of the rotary kiln 20, kiln fuel $F_{kiln}$ is input, in the form of gas, oil, or pulverized solid fuel, producing a large concentric flame in the lower part of the rotary kiln 20. As hotmeal moves under the flame, it reaches its peak temperature, before dropping out of the rotary kiln 20 as clinker C into the cooler 30. Cooling air is provided by air blowers 32 and is drawn first through the cooler 30 and then exhausted through the rotary kiln 20 for combustion of the kiln fuel $F_{kiln}$. A portion of the cooler exhaust $E_{cooler}$ is directed through a tertiary air pipe 31 for combustion in a precalciner 12.

In the following, reference will be made to "raw meal" and "hotmeal". In the context of the present disclosure, the term "raw meal" refers to the raw ingredients provided into the cement kiln 100 at the input to the cement preheater 10. The raw meal includes a ground mixture of limestone, clay or shale used for generating cement clinker. On the other hand, the term "hotmeal" refers to the ingredients of the raw meal at any point after drying and before clinkering. In other words, the term "hotmeal" refers to the partially- or completely-calcined kiln feed material present in the cement preheater 10, at any location in the cement preheater 10 between a point of initial drying of the raw meal and the point of entry into the rotary kiln 20. Further, in the context of the present disclosure, the term "clinker" refers to the solid material produced by the cement kiln 100, as a result of sintering of the hotmeal provided to the rotary kiln 20.

When referring to a quantity of the raw meal (e.g. a raw meal sulphur content, raw meal alkali content, etc.), the quantity is to be thought of as a value measured or estimated from a sample of raw meal taken before entry into the cement preheater. Similarly, when referring to a quantity of the hotmeal (e.g. a hotmeal sulphur content, hotmeal alkali content, etc.), the quantity is thought of as a value measured or estimated from a sample of hotmeal taken from any point in the cement preheater 10, including but not limited to the cement cyclones 11, the precalciner 12 and any ducting therebetween. Further, when referring to a quantity of the clinker (e.g. a clinker sulphur content, etc.), the quantity is thought of as a value measured or estimated from a sample of clinker taken at any point from the exit of the rotary kiln 20. That is, the clinker may be sampled prior to being cooled by the cooler 30, or after exiting the cooler 30.

The conventional cement kiln 100 shown in Fig. 1 further includes a cement preheater 10. In the context of the present disclosure, the cement preheater 10 is a gas-suspension preheater, whereby hot exhaust gas from the rotary kiln 20 is passed through a suspension of hotmeal so as to preheat the hotmeal. Additionally, the cement preheater 10 causes the hotmeal to be calcinated. The cement preheater 10 allows for high efficiency of the cement kiln 100 through heat transfer from the exhaust gas to the hotmeal. The hotmeal preheating takes place in a number of cement cyclones 11 - the cement preheater 10 as exemplarily shown in the figures includes four cement cyclones 11 cascaded in series. However, the cement preheater 10 may include any number of cement cyclones 11, arranged in series, in parallel, or in series-parallel combinations.

To further improve efficiency, the cement preheater 10 as exemplarily shown may optionally include a cement precalciner 12. Hot air $E_{cooler}$ exhausted from the cooler 30 is provided to the cement precalciner 12, as well as a precalciner fuel $F_{calc}$. Typically, the precalciner fuel $F_{calc}$ includes pulverised coal. By combusting additional fuel in the precalciner 12, the hotmeal is further heated to a higher temperature than is possible with kiln exhaust only, allowing the hotmeal to be calcinated to a higher level and improving throughput.

By implementing a cement preheater 10 with cement cyclones 11 and cement precalciner 12 for preheating the raw meal, a hotmeal may be prepared having a temperature of 700°C to 900°C with up to 90% calcination prior to entering the rotary kiln 20.

However, challenges arise when blockages form in the cement preheater. For example, blockage of the narrow passages and ducts of the cement preheater 10, particularly of a cement cyclone 11, causes reduced throughput of the cement preheater 10, and potentially a complete shutdown of the cement kiln 100. Significant monetary losses can be incurred by unscheduled downtime, therefore the monitoring of blockages in the cement preheater 10 is a major concern.

There are two types of blockage scenarios possible for a cement preheater. Firstly, a mechanical blockage may occur, wherein a passage of the cement preheater 10 gets obstructed due to failure of a duct or refractory lining. Secondly, chemical blockages may occur due to agglomeration. In the present disclosure, the aspects of blockage due to chemical agglomeration will be addressed.

The presence of molten alkali chlorides and sulphur in petcoke and other combustible fuel, which is introduced into the cement preheater 10 as exhaust gas from the rotary kiln 20 and/or precalciner 12, causes agglomeration of sulphur and alkali compounds. During the preheating process in the cement preheater 10, and particularly in a cement cyclone 11, volatile contents of the hotmeal and exhaust gas (e.g. compounds of sulphur, chlorine, and alkali) is oxidized, which tend to condense and accumulate on colder areas in the cement preheater 10, liberating various oxides including sulphur.

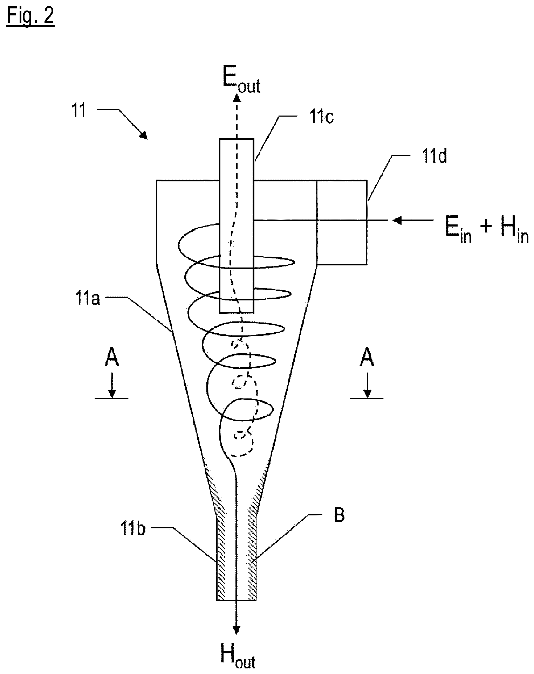

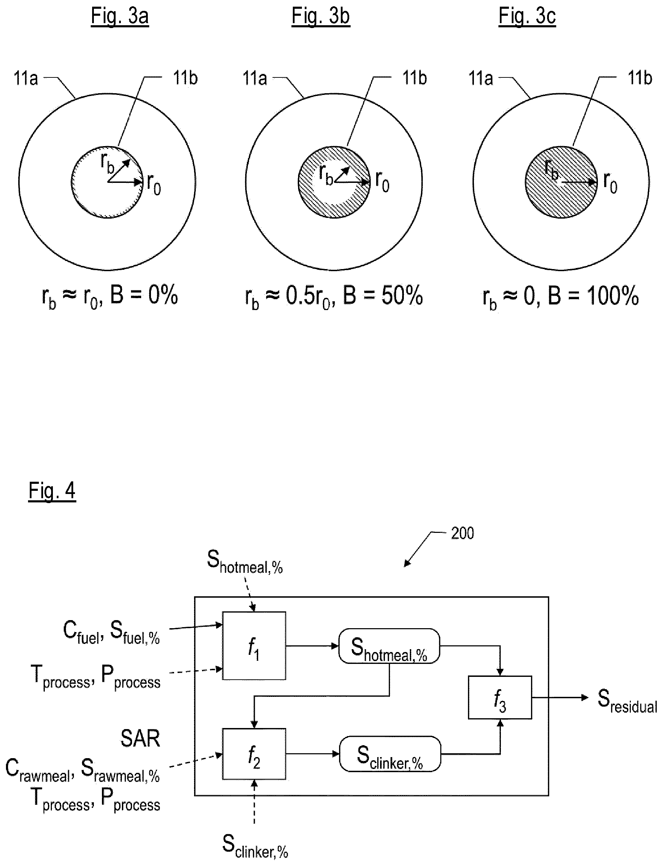

Reference will now be made to Fig. 2, which shows a schematic side section view of a cement cyclone 11, which form part of the cement preheater 10. Further reference is made to Figs. 3A-3C, which show a top section view of the cement cyclone 11 along section A-A. The cement cyclone 11 includes a cyclone barrel 11a, a cyclone exit 11b, a cyclone exhaust 11c and a cyclone entry 11d. The cyclone entry 11d is arranged tangentially to the cyclone barrel 11a, such that an entering flow of exhaust gas $E_{in}$ with suspended hotmeal $H_{in}$ enters the cyclone 11 and forms a vortex. Heat is transferred from the hot exhaust gas to the suspended hotmeal. The vortex causes solid particles of hotmeal to be forced out of suspension, dropping downwards out of the cyclone 11 through cyclone exit 11b, while the clean exhaust gas $E_{out}$ exits the cyclone 11 via the cylone exhaust 11c. Certain passages or ducts of the cement cyclone 11 may be particularly prone to blockage - as exemplarily shown in the figure, material is deposited on the walls of the narrow cyclone exit 11b causing a blockage B.

In the context of the present disclosure, a blockage B may be defined as a percentage of the duct or passage which is blocked by agglomerated compounds. For example, the cyclone outlet 11b shown in Fig. 3A has a small amount of compound agglomerated thereto, and the nominal radius of the cyclone exit $r_0$ is approximately equal to the radius of the blockage $r_d$. This duct is considered to be substantially unblocked, and the blockage B is deemed to be at or near B = 0%. Fig. 3B exemplarily shows a partially blocked cyclone exit 11b, whereby the radius of the blockage $r_d$ is approximately half of the nominal radius of the cyclone exit $r_0$. This duct is considered to be partially blocked, and the blockage B is deemed to be at or near B = 50%. Finally, Fig. 3C exemplarily shows a cyclone exit 11b which is completely or almost completely blocked, wherein the radius of the blockage $r_b$ is at or near zero. This duct is considered to be fully blocked, and the blockage B is deemed to be at or near B = 100%.

Blockages may result from the combined action of recirculating materials (alkalis, chlorides, sulphates) and intermediate compounds from the decomposition of raw meal that tend to stick together. Since agglomeration mainly occurs due to the formation of alkali sulphates, sulphur becomes the major cause of agglomeration. Blockages in the cyclone area or hotmeal ducts can be caused by the gradual closure of narrow cross-sections or through a sudden fall of dislodged compounds which, for example, closes a duct, passage or cyclone outlet. The frequency of cyclone blockages is strongly influenced by many factors, including kiln design, cement preheater design, raw meal quality, fuel quality and process parameters.

Sulphur compounds originate primarily from the kiln fuel and exit the cement kiln as part of the produced clinker. However, a significant amount of volatile sulphur remains in the cement preheater 10, continually condensing and evaporating due to periodic heating and cooling. The recirculation of these contents causes an amount of "residual sulphur" which is contained in the cement preheater 10. This amount of residual sulphur may recirculate throughout the cement preheater 10 and continue to deposit sulphur compounds on the walls of the cement preheater 10, particularly the cyclones 11, leading to agglomeration and chemical blockage. In the context of the present disclosure, the term "residual sulphur" refers to a total amount of volatile sulphur compounds present and circulating in the cement preheater 10. In order to monitor and evaluate blockages and the level of agglomeration in the cement preheater 10, it is an object of the present disclosure to provide a method and apparatus for evaluating the amount of residual sulphur. However, due to the high temperatures of 700°C to 1000°C in the region of the cement preheater, the incorporation of a physical sensor for detecting residual sulphur is typically impractical. Therefore, a method for evaluating residual sulphur through estimation, modelling and/or machine learning is proposed.

According to a first aspect of the present disclosure, a method for evaluating residual sulphur in a cement preheater of a cement kiln is provided. The method includes determining values of fuel sulphur content and fuel rate consumption of a fuel being provided to the cement kiln, determining a value of hotmeal quality of a hotmeal being provided to the cement kiln, determining a value of clinker sulphur content of a clinker being produced by the cement kiln, and evaluating the residual sulphur in the cement preheater using a sulphur evaluation unit, wherein the residual sulphur is based on the values of the fuel sulphur content, the fuel rate of consumption, the hotmeal quality and the clinker sulphur content.

With embodiments of the present disclosure, the evaluation of the amount of residual sulphur can be carried out with a model which considers various sulphur-related quantities. Based on these quantities, a reliable estimation of the amount of residual sulphur can be obtained without the presence of a physical sensor for sensing residual sulphur within the harsh environment of the cement preheater 10.

The method for evaluating residual sulphur is carried out on a sulphur evaluation unit. The sulphur evaluation unit may include a controller, a microprocessor, a programmable logic controller (PLC), or a digital signal processor (DSP). Particularly, the sulphur evaluation unit may include a processing element, at least one input and at least one output, such that a data processing operation is performed on the at least one input and output to the at least one output. The sulphur evaluation unit may further include at least one storage means, which may include random access memory (RAM), read-only memory (ROM) and external data storage means such as hard disks, flash storage or network-attached storage. The sulphur evaluation unit may further include a wired or wireless data connection for interfacing with a data network.

The method for evaluating residual sulphur is based on a number of quantities, parameters or values which are relevant to the amount of residual sulphur. The quantities may be categorised as a group of values relating to inputs of sulphur compounds, a group of values relating to outputs of sulphur compounds, and a group of values relating to sulphur compounds contained in the cement preheater.

The group of values relating to inputs of sulphur compounds include quantities and parameters of the fuel, and optionally the raw meal. Although the kiln fuel is the primary source of sulphur compounds, the quality of the raw meal input into the cement preheater may also be relevant if the raw meal contains a significant amount of sulphur. The method for evaluating residual sulphur according to the present disclosure is based on values of fuel sulphur content $S_{fuel,\%}$ and fuel rate of consumption $C_{fuel}$ so as to determine the total amount of sulphur being input into the system. The fuel sulphur content $S_{fuel,\%}$ may be determined by measurements obtained from samples of the fuel or from data provided by the fuel supplier, and may be either manually input by an operator or obtained from an attached data storage. The fuel rate of consumption $C_{fuel}$ may be determined directly by a sensor attached to the sulphur evaluation unit, may be obtained from a kiln management system or an attached data storage, or may be provided manually by an operator. Optionally, the method for evaluating residual sulphur may be further based on at least one of the raw meal sulphur content $S_{rawmeal,\%}$, the raw meal alkali content $A_{rawmeal,\%}$, and a raw meal rate of consumption $C_{rawmeal}$.

The group of values relating to outputs of sulphur compounds may include quantities and parameters of the clinker, and optionally the exhaust gas exiting the cement preheater. The clinker produced by the cement kiln is the primary output of sulphur compounds, so the evaluation of residual sulphur is based on the clinker sulphur content $S_{clinker,\%}$. The clinker sulphur content $S_{clinker,\%}$ may be determined by measurements obtained from samples of the clinker, and may be either manually input by an operator or obtained from an attached data storage. Further, some volatile sulphur compounds may be contained in the exhaust gas which exits the cement preheater via the exhaust $E_{out}$. Thus, the evaluation of residual sulphur may be optionally further based on at least one of the exhaust gas sulphur content and/or an exhaust gas temperature.

The group of values relating to the sulphur contained in the cement preheater may include quantities and parameters of the hotmeal, and optionally process parameters of the cement preheater. The method for evaluating residual sulphur is based on a hotmeal quality $Q_{hotmeal}$. In the context of the present disclosure, a hotmeal quality may be a sulphur- or alkali-related quantity of the hotmeal, which may be obtained by sampling and measuring the sulphur content of the hotmeal, or may be obtained based on empirical data, simulation data, or based on the values obtained for the fuel sulphur content $S_{fuel,\%}$ and fuel rate of consumption $C_{fuel}$. The value may be provided to the sulphur evaluation unit by either manual input by an operator, by being obtained from an attached data storage, or may be determined by the sulphur evaluation unit based on other parameters. Optionally, the method for evaluating residual sulphur may be further based on process parameters, such as at least one temperature at a predetermined location in the cement preheater, and/or at least one pressure at a predetermined location in the cement preheater. The process parameters in the cement preheater may influence the amount of residual sulphur which is retained in the hotmeal, and the amount of residual sulphur which ultimately deposits on the walls of the cement preheater.

According to an embodiment, which may be combined with other embodiments described herein, the value of hotmeal quality $Q_{hotmeal}$ includes at least one of a hotmeal sulphur content $S_{hotmeal,\%}$ and a hotmeal alkali content $A_{hotmeal,\%}$. The hotmeal sulphur content $S_{hotmeal,\%}$ corresponds to the amount of sulphur compounds present in a sample of hotmeal taken from a predetermined position in the cement preheater. Similarly, the hotmeal alkali content $A_{alkali,\%}$ corresponds to the amount of alkali compounds present in a sample of hotmeal taken from a predetermined position in the cement preheater. Particularly, the position in the cement preheater from which the respective sample is taken may be at the exit of the cement preheater where the hotmeal enters the rotary kiln (i.e. where the hotmeal is fully calcinated) or at an intermediate position in the cement preheater where volatile sulphur compounds are circulating in the cement preheater.

Optionally, the method for evaluating residual sulphur may be further based on a sulphur-alkali ratio (SAR), which may be defined as the amount of sulphur compounds present in the raw meal divided by the amount of alkali compounds present in the raw meal. The sulphur-alkali ratio (SAR) has an influence on the amount of sulphur compounds which are retained in the hotmeal and which exit the system as clinker. For example, if the SAR is above 1, then the amount of sulphur compounds is in excess, resulting in an increase in residual sulphur retained in the cement preheater as volatile compounds. On the other hand, if the SAR is at 1 or below, the amount of excess sulphur compounds is neutralized, resulting in a lower amount of sulphur retained in the cement preheater as volatile compounds.

Although the method for evaluating residual sulphur is allowed to function by providing static values for the various quantities relating to the fuel, hotmeal and clinker, the accuracy and reliability of the evaluation of the residual sulphur is improved by periodically providing updated values for such quantities. By providing periodic measurements, fluctuations in fuel quality, fuel type or raw meal quality can be accounted for.

According to an embodiment, which may be combined with other embodiments described herein, the method for evaluating residual sulphur may further include measuring at least one of the fuel sulphur content, the hotmeal quality and the clinker sulphur content, such that the measurement is performed periodically in time. For example, the kiln fuel may be sampled periodically, and a new value for the fuel sulphur content $S_{fuel,\%}$ may be determined and provided to the sulphur evaluation unit.

Between the time of entry of the raw meal into the cement preheater and the time of exit of the produced clinker, the processing time can be in the range of approximately 30 minutes. It follows that a change or variation in raw meal quality may be realised at the clinker output approximately 30 minutes later. However, typically the time between sampling and measuring of quality data for the raw meal sulphur content $S_{rawmeal,\%}$, hotmeal sulphur content $S_{hotmeal,\%}$, clinker sulphur content $S_{clinker,\%}$ and/or fuel sulphur content $S_{fuel,\%}$ is much longer than 30 minutes, and may be up to 8 hours. Meanwhile, values such as fuel rate of consumption $C_{fuel}$, raw meal rate of consumption $C_{rawmeal}$ and process temperature/pressure may be obtained in real-time, or at a significantly shorter time period as compared to sampled values. Further, the sampled values may be measured at different, non-corresponding times, depending on laboratory workload.

As a result of the differing measurement frequencies, it may become challenging to relate certain values of the fuel, raw meal or hotmeal to other values of the clinker. To alleviate such challenges, the sampling frequency may be increased through temporal upsampling. According to an embodiment, which may be combined with other embodiments described herein, at least one of the periodically-measured values are upsampled, particularly temporally upsampled. The upsampling may include any method or model known in the art, including regression modelling, interpolation or extrapolation. The upsampling may be further based on empirical data, simulation data, or a combination thereof. In the present disclosure, the upsampling may be implemented by a controller, a microprocessor, a filter, or a digital signal processor (DSP). Particularly, the upsampling may be implemented by a multi-rate digital signal processor (DSP), which may be contained within the sulphur evaluation unit 200.

For example, at least one of the hotmeal sulphur content $S_{hotmeal,\%}$, the clinker sulphur content $S_{clinker,\%}$, the fuel sulphur content $S_{fuel,\%}$, and/or the raw meal sulphur content $S_{rawmeal,\%}$ may be sampled and measured at a predetermined time interval, and the respective values are upsampled to provide values which are estimated at a shorter time interval. Further, different values may be sampled and measured at different, non-corresponding points in time, and upsampled so as to provide a respective corresponding data point for every value at the same predetermined point in time. For example, the respective time periods of the upsampled values may be at most 1 hour, particularly at most 30 minutes, and more particularly at most 1 minute. In a preferred embodiment, the respective time periods of the upsampled values are 30 minutes. Further, the respective time periods of the upsampled values may be adjusted to correspond to a time period of values detected by physical sensors configured for measuring kiln parameters, cement preheater parameters, fuel and/or raw meal rate of consumption, etc. In a preferred embodiment, all values, quantities and parameters which are to be either measured directly by physical sensors or which are to be upsampled are set to the same time period of at most 1 hour, particularly at most 30 minutes, and more particularly at most 1 minute, or may be preferably adjusted to time period of 30 minutes.

As an example for implementing the methods according to the present disclosure, an exemplary implementation of the method for evaluating residual sulphur is shown in Fig. 4. The exemplary implementation includes a sulphur evaluation unit 200 which is configured for evaluating the residual sulphur $S_{residual}$ based on the input quantities. Inputs which are deemed optional are shown with dashed lines.

In the example implementation, the sulphur evaluation unit 200 is provided with, at a minimum, a value of the fuel rate of consumption $C_{fuel}$ and a value of the fuel sulphur content $S_{fuel,\%}$. A first block function f1 is configured for determining a hotmeal sulphur content $S_{hotmeal,\%}$ based at least on the fuel rate of consumption $C_{fuel}$ and the fuel sulphur content $S_{fuel,\%}$. Optionally, the first block function f1 may further determine the hotmeal sulphur content $S_{hotmeal,\%}$ based on at least one process temperature $T_{process}$ and/or at least one process pressure $P_{process}$. The first block function $f_1$ implements a model, which may include a multidimensional, nonlinear model or a machine learning model, which determines the hotmeal sulphur content $S_{hotmeal,\%}$ based at least on the rate of sulphur entering the system from the fuel. Optionally, values for the hotmeal sulphur content $S_{hotmeal,\%}$ may be periodically provided to the sulphur evaluation unit 200 to the first block function $f_1$, based on measurements taken from samples of hotmeal. These sampled measurements of hotmeal sulphur content $S_{hotmeal,\%}$ may subsequently be interpolated or upsampled in the model contained in first block function $f_1$.

After determining a value of hotmeal sulphur content $S_{hotmeal,\%}$, the sulphur evaluation unit 200 is configured to determine a value of clinker sulphur content $S_{clinker,\%}$ via second block function $f_2$. The clinker sulphur content $S_{clinker,\%}$ is based on the hotmeal sulphur content $S_{hotmeal,\%}$ and one or more optional parameters. Similar to first block function $f_1$, second block function $f_2$ implements a model, which may include a multidimensional, nonlinear model or a machine learning model. Optionally, values for the clinker sulphur content $S_{clinker,\%}$ may be periodically provided to the sulphur evaluation unit 200 to the second block function $f_2$, based on measurements taken from samples of clinker. These sampled measurements of clinker sulphur content $S_{clinker,\%}$ may subsequently be interpolated or upsampled in the model contained in second block function $f_2$.

Finally, the sulphur evaluation model 200 evaluates the residual sulphur $S_{residual}$ in the cement preheater based on the hotmeal sulphur content $S_{hotmeal,\%}$ and the clinker sulphur content $S_{clinker,\%}$ via third block function f3. In a most simplified example, third block function f3 subtracts the clinker sulphur content $S_{clinker,\%}$ from the hotmeal sulphur content $S_{hotmeal,\%}$ to determine the residual sulphur $S_{residual}$. Alternatively, the third block function f3 may include a multidimensional, nonlinear model or a machine learning model.

According to an embodiment, which may be combined with other embodiments described herein, the sulphur evaluation unit 200 may be configured to evaluate the residual sulphur $S_{residual}$ by a parameterized function having adjustable model parameters, wherein the method for evaluating residual sulphur further includes adjusting the model parameters. Particularly, the sulphur evaluation unit 200 may be configured to implement a machine learning model for at least one of the determining a value of hotmeal quality $Q_{hotmeal}$ and evaluating the residual sulphur in the cement preheater. In other words, the sulphur evaluation unit 200 may be configured to implement a machine learning model for at least one of the block functions $f_1$, $f_2$ and $f_3$.

Machine learning techniques train models to accurately make evaluations, estimations and predictions on data fed into the models. During a learning phase, the models are developed against a training dataset of inputs to optimize the models to correctly predict the output for a given input. Generally, the learning phase may be supervised, semi-supervised, or unsupervised, indicating a decreasing level to which the "correct" outputs are provided in correspondence to the training inputs. **In** a supervised learning phase, all of the outputs are provided to the model and the model is directed to develop a general rule or algorithm that maps the input to the output. **In** contrast, in an unsupervised learning phase, the desired output is not provided for the inputs so that the model may develop its own rules to discover relationships within the training dataset. **In** a semi-supervised learning phase, an incompletely labelled training set is provided, with some of the outputs known and some unknown for the training dataset.

Models may be run against a training dataset for several iterations to refine its results. For example, in a supervised learning phase, a model is developed to predict the output for a given set of inputs, and is evaluated over several iterations to more reliably provide the output that is specified as corresponding to the given input for the greatest number of inputs for the training dataset. In another example, for an unsupervised learning phase, a model is developed to cluster the dataset into groups, and is evaluated over several iterations as to how consistently it places a given input into a given group and how reliably it produces the group clusters across each iteration. Once an iteration is run, the models are evaluated and the values of their variables are adjusted to attempt to better refine the model in an iterative fashion. One of ordinary skill in the art will be familiar with various machine learning algorithms that may be applied to the present disclosure, including linear regression, random forests, decision tree learning, neural networks, deep neural networks, etc.

Some machine learning models, which may be adapted for embodiments of the present disclosure, are implemented with or are trained by a neural network (e.g., deep learning, deep convolutional, or recurrent neural network), which comprises a series of "neurons", such as Long Short Term Memory (LSTM) nodes, arranged into a network. A neuron is an architectural element used in data processing and artificial intelligence, particularly machine learning, that includes memory that may determine when to "remember" and when to "forget" values held in that memory based on the weights of inputs provided to the given neuron. Each of the neurons used herein are configured to accept a predefined number of inputs from other neurons in the network to provide relational and sub-relational outputs for the content of the frames being analyzed. Individual neurons may be chained together and/or organized into tree structures in various configurations of neural networks to provide interactions and relationship learning modeling for how each of the frames in an utterance are related to one another.

For example, an LSTM serving as a neuron includes several gates to handle input vectors, a memory cell, and an output vector. The input gate and output gate control the information flowing into and out of the memory cell, respectively, whereas forget gates optionally remove information from the memory cell based on the inputs from linked cells earlier in the neural network. Weights and bias vectors for the various gates are adjusted over the course of a training phase, and once the training phase is complete, those weights and biases are finalized for normal operation. One of skill in the art will appreciate that neurons and neural networks may be constructed programmatically (e.g., via software instructions) or via specialized hardware linking each neuron to form the neural network.

The machine learning model to be implemented for any one of block functions $f_1$, $f_2$ and/or $f_3$ may be an artificial neural network model. The model may include a plurality of input neurons, at least one layer of a plurality of hidden neurons, and a plurality of output neurons. For example, an artificial neural network for implementing the model of any one of block functions $f_1$, $f_2$ and/or $f_3$ may include 3 layers of hidden neurons, with each layer having 16 neurons. Each neuron of a layer of hidden neurons may be fully connected or partially connected to each neuron of another layer of hidden neurons. However, the present disclosure is not limited thereto, and a model having any number of neurons and/or layers of neurons may be implemented.

According to embodiments of the present disclosure, a sensor device for evaluating residual sulphur in a cement preheater of a cement kiln is provided. The sensor device includes a determining unit configured for determining values of fuel sulphur content $S_{fuel,\%}$, fuel rate of consumption $C_{fuel}$, hotmeal quality $Q_{hotmeal}$ and clinker sulphur content $S_{clinker,\%}$, and a sulphur evaluation unit configured for evaluating the residual sulphur according to methods of the first aspect of the present disclosure.

With the absence of a physical sensor for sensing residual sulphur, the implementation of the method for evaluating residual sulphur may be realised as a sensor device configured for carrying out the method. In other words, the sensor device may be a so-called "soft sensor", in which a signal is generated corresponding to the residual sulphur based on the use of software, modelling or signal processing in relation to quantities, parameters and values which are input to the "soft sensor". In the context of the present disclosure, the term "sensor device" may be used interchangeably with the term "soft sensor".

The determining unit may include any means for determining the values upon which the residual sulphur $S_{residual}$ is based. For example, for values which may be measured by physical sensors, such as a fuel rate of consumption $C_{fuel}$, the determining unit may be configured for receiving a signal from said physical sensor, and further for providing the value to the sulphur evaluation unit. As another example, for values which may be determined based on a model or function, such as hotmeal quality $Q_{hotmeal}$, the determining unit may include a controller, microcontroller or digital signal processor (DSP) configured for determining the value. As yet another example, for values which may be obtained from an external storage means, the determining unit may be configured for retrieving data from an external storage means. As yet another example, for values which may be input by a human operator, the determining unit may include an input means configured for receiving input from a human operator. The determining unit may be a separate unit, or may be included as part of the sulphur evaluation unit.

According to an embodiment, which may be combined with other embodiments described herein, the determining unit is configured for at least one of the following: receiving at least one of the values from a manual operator, receiving at least one of the values from a data store, and measuring at least one of the values.

The determining unit may be further configured for upsampling at least one of the values, particularly temporally upsampling at least one of the values.

By providing an evaluation of the residual sulphur $S_{residual}$ in the cement preheater, it is now possible to further provide methods and systems for monitoring and evaluating blockages of the cement preheater, and to implement further actions for controlling, limiting or remedying blockages. According to a second aspect of the present disclosure, a method for evaluating blockage in a cement preheater is provided. The method includes evaluating the residual sulphur in the cement preheater according to a method according to embodiments of the first aspect of the present disclosure, determining an agglomeration rate of sulphur compounds agglomerating on an inner surface of the cement preheater based on the residual sulphur, and evaluating a level of blockage in at least one predetermined pathway of the cement preheater using a blockage evaluation unit, wherein the level of blockage is based on the agglomeration rate.

The method for evaluating blockage in the cement preheater includes first evaluating the residual sulphur in the cement preheater. The evaluation of residual sulphur may be carried out according to any of the aspects and embodiments described in the present disclosure.

Upon obtaining the residual sulphur $S_{residual}$, an evaluation of the rate of agglomeration of sulphur compounds on the inner walls of the cement preheater can be performed. Since agglomeration build-up is a relatively slow but continuous process, a rate of agglomeration and estimated level of blockage, and subsequently an estimated time until blockage, may be determined based on a linear or non-linear gradient model, wherein the gradient model is prepared based on any one of empirical data, simulation data or a combination thereof.

In a simplistic example, the rate of agglomeration may be determined based on the residual sulphur $S_{residual}$ and a lookup table or gradient model. Once the rate of agglomeration has been determined, a further lookup table or gradient model based on empirical data prepared in view of the design and geometry of the cement preheater may be used to determine an estimated time to complete blockage.

To evaluate a level of blockage, an initial measurement of the level of blockage may be performed during a period of maintenance and stored as an initial level of blockage. The current level of blockage $B_{current}$ can then be determined based on the initial level of blockage $B_1$ and the rate of agglomeration. For example, in a very simplistic linear gradient version of the blockage determination model, at the previous maintenance period a level of blockage in a predetermined pathway of the cement preheater may have been measured at $B_1 = 20\%$, and a rate of agglomeration was evaluated after 20 days at 0.5% per day, the current level of blockage $B_{current} = 30\%$, and the estimated time until blockage is 140 days.

However, the rate of agglomeration is typically non-linear in practice, and may depend heavily on the design of the cement preheater, the process parameters, and the type and quality of the fuel and/or raw meal being provided. Therefore, embodiments of the the present disclosure seek to implement multidimensional non-linear models, parameterized function models and/or machine learning models to determine the rate of agglomeration, level of blockage and estimated time until blockage. According to an embodiment, which may be combined with other embodiments described herein, the blockage evaluation unit is further configured for at least one of the determining the rate of agglomeration and the evaluating the level of blockage by a parameterized function having adjustable model parameters, wherein the method for evaluating blockage preferably further includes adjusting the model parameters. Particularly, the blockage evaluation unit may be configured to implement a machine learning model for evaluating at least one of the agglomeration rate, a level of blockage and an estimated time until blockage.

In a similar manner to the method for evaluating residual sulphur described in the first aspect, the machine learning model to be implemented for evaluating a level of blockage may be an artificial neural network model. The model may include a plurality of input neurons, at least one layer of a plurality of hidden neurons, and a plurality of output neurons. For example, an artificial neural network for implementing the model of any one of block functions $f_1$, $f_2$ and/or $f_3$ may include 3 layers of hidden neurons, with each layer having 16 neurons. Each neuron of a layer of hidden neurons may be fully connected or partially connected to each neuron of another layer of hidden neurons. However, the present disclosure is not limited thereto, and a model having any number of neurons and/or layers of neurons may be implemented.

Since the design of a cement kiln, particularly the design of a cement preheater, can influence the rate at which sulphur compounds agglomerate, it may be necessary to configure the model for evaluating blockages based on empirical data, simulation data, or combinations thereof. The blockage evaluation method, particularly the model for evaluating an agglomeration rate and/or a level of blockage, may further include an initialization, calibration and/or training in which the adjustable model parameters are set and/or adjusted.

For implementing the blockage evaluation method on existing cement kilns, the initialization, calibration and/or training of the models implemented by the blockage evaluation unit, particularly one or more machine learning models, may be based on maintenance data. According to embodiments, which may be combined with other embodiments described herein, the adjustable model parameters are adjusted based on training data including one of a maintenance-to-maintenance measurement or a maintenance-to-blockage measurement.

In the use of a maintenance-to-maintenance measurement, the rate of agglomeration is based on a first level of blockage $B_1$ measured at a first maintenance interval $t_1$ and a second level of blockage $B_2$ measured at a second maintenance interval $t_2$. In this case, the evaluation of the rate of agglomeration may be initialized, calibrated and/or trained according to the following:

$$Rate\ of\ agglomeration = \frac{\Delta B}{\Delta t} = \frac{(B_2 - B_1)}{(t_2 - t_1)} \qquad \dots (1)$$

In the use of maintenance-to-blockage measurement, the rate of agglomeration is based on a first level of blockage $B_1$ measured at a first maintenance interval $t_1$ and a level of complete blockage at a second interval $t_2$. The second interval may be a time at which the cement kiln has been shut down due to blockage of the cement preheater. In this case, the evaluation of the rate of agglomeration may be initialized, calibrated and/or trained according to the following:

$$Rate\ of\ agglomeration = \frac{\Delta B}{\Delta t} = \frac{100 - B_1}{(t_2 - t_1)} \qquad \dots (1)$$

In the above, the level of blockage B is in a range of 0%, representing a completely unblocked pathway with no agglomeration, and 100%, representing a completely blocked pathway which is fully agglomerated (see also Figs. 3A-3C).

For implementing the blockage evaluation method on new and existing cement kilns, the initialization, calibration and/or training of the models implemented by the blockage evaluation unit, particularly one or more machine learning models, may be based on a surrogate model. In the context of the present disclosure, the term "surrogate model" may be referred to as a baseline model by which the method of evaluating a blockage may be initialized so as to ensure initial operation. The surrogate model may then be iteratively improved based on empirical data collected during continued operation of the cement kiln. By implementing a surrogate model, a new cement plant which implements a blockage evaluation method may be brought into service sooner, or an existing cement plant may be rapidly retrofitted with a new blockage evaluation implementation, with the reliability and accuracy of the blockage evaluation being iteratively improved as the cement kiln is operated.

The surrogate model may be based on data conceived in the design of the cement preheater, such as geometrical data, flow data, material data, heat transfer data, etc. The surrogate model may further include data relating to process parameters by which the cement preheater is to be operated. Optionally, the surrogate model may be based on empirical data measured on a different cement kiln or cement preheater, which may have similarities in design.

The surrogate model may be further based on simulation data. For example, during the design of a new cement preheater, computational fluid dynamics (CFD) simulations may be used to optimise flow of gases and hotmeal through the cement preheater. The data generated by such simulations may also be used to train a machine learning model which is configured for evaluating blockages. According to an embodiment, which may be combined with other embodiments described herein, the adjustable model parameters are adjusted based on training data comprising at least one computational fluid dynamics (CFD) simulation of the cement preheater, preferably wherein the computational fluid dynamics (CFD) simulation is iterative.

However, the present disclosure is not limited only to computational fluid dynamics (CFD) simulations. For example, the surrogate model and/or training data for training the surrogate model may further include simulation data from heat transfer simulations.

The method for evaluating blockage is carried out on a blockage evaluation unit. The blockage evaluation unit may include a controller, a microprocessor, a programmable logic controller (PLC), or a digital signal processor (DSP). Particularly, the blockage evaluation unit may include a processing element, at least one input and at least one output, such that a data processing operation is performed on the at least one input and output to the at least one output. The blockage evaluation unit may further include at least one storage means, which may include random access memory (RAM), read-only memory (ROM) and external data storage means such as hard disks, flash storage or network-attached storage. The blockage evaluation unit may further include a wired or wireless data connection for interfacing with a data network.

Note that the blockage evaluation unit for evaluating blockage and the sulphur evaluation unit for evaluating residual sulphur may be separate units, or may be the same unit.

The blockage evaluation unit is configured to evaluate a level of blockage in at least one predetermined pathway of the cement preheater. In the context of the present disclosure, the term "predetermined pathway" refers to any duct, pipe, opening, or flow element provided in the cement preheater which may become blocked. In particular, a "predetermined pathway" may refer to a specific duct, pipe, opening or flow element which is to be monitored by the blockage evaluation unit.

The method of evaluating a blockage in a cement preheater according to aspects and embodiments described herein may be further extended to include measures to limit, control and/or prevent blockage from occurring, based on at least one of the level of blockage, the agglomeration rate, or the amount of residual sulphur in the cement preheater.

According to an embodiment, which may be combined with other embodiments described herein, the method for evaluating blockage may further include generating a signal indicating whether at least one of the residual sulphur and the level of blockage has exceeded a threshold value. A threshold value may be set based on the maximum allowable value. Optionally, intermediate threshold values may be set based on values indicating a warning threshold, an action threshold, a critical threshold and/or a kiln shutdown threshold.

The generation of a signal indicating the exceedance of a threshold value may be used for many purposes. For example, at a basic level, the exceeding of a threshold value may sound an alarm or light an indicator light indicating to an operator that a blockage may occur. As a further example, the signal indicating exceedance of a threshold value may be transmitted to a data network for datalogging purposes or for sending automated messages.

In the present disclosure, a signal indicating an exceedance of a threshold value may be used for remedying or controlling the agglomeration of sulphur compounds and blockage of a cement preheater. The signal may trigger or indicate to an operator that a cleaning event is to be carried out, where the blockage may be cleared by mechanical means. For example, the signal may trigger the scheduling of a maintenance period by which the cement preheater is cleaned. The signal may instead initiate or schedule an in-situ cleaning operation, such as an air blast wherein compressed air is injected into the passage at which a suspected blockage is present, dislodging agglomerated compounds and clearing the blockage.

Alternatively, the signal may trigger or indicate to an operator that a correction to the sulphur-alkali ratio (SAR) is to be carried out. To neutralize excess residual sulphur and its effects, i.e. to control the agglomeration of sulphur compounds, alkali may be added to the raw meal feed in-situ so that the excess residual sulphur is instead retained in the hotmeal, exiting the process as clinker. Typically, the addition of alkali is managed by keeping sulphur-alkali ratio (SAR) at or near 1.

According to an embodiment, which may be combined with other embodiments described herein, the method for evaluating blockage further includes generating a command signal to initiate at least one of the following: scheduling or initiating a planned cleaning event, wherein the blockage is mechanically cleared, particularly by an air blast, and scheduling or initiating an SAR correction event, wherein the sulphur-alkali ratio (SAR) is corrected by introducing alkali into the cement preheater.

After the generation of a signal which initiates a cleaning event or an SAR correction, the blockage evaluation unit may further perform a correction to at least one of the level of blockage, the agglomeration rate or the residual sulphur. For example, a model which estimates the amount of blockage cleared by an air blast may be implemented to estimate a level of blockage after the air blast. As a further example, a model which estimates a reduction of residual sulphur caused by an SAR correction may be implemented to evaluate the residual sulphur after a predetermined amount of alkali is added to the system.

According to an embodiment, which may be combined with other embodiments described herein, the sensor device may further include a network interface for connecting the sensor device to a data network, in particular a global data network. In this embodiment, the sensor device is operatively connected to the network interface for carrying out commands received from the data network. The commands may include receiving at least one of the values, i.e. the parameters described above for evaluating residual sulphur. The commands may further include carrying out a command received from the data network. In this case, the device/controller is adapted for carrying out the task in response to the control command. The commands may include a status request. In response to the status request, or without prior status request, the sensor device may be adapted for sending a status information to the data network. Particularly, the sensor device may be adapted for sending status information to the network interface, and the network interface is then adapted for sending the status information over the data network. The commands may include an update command including update data. In this case, the device/controller is adapted for initiating an update in response to the update command and using the update data.

The data network may be an Ethernet network using TCP/IP such as LAN, WAN or Internet. The data network may comprise distributed storage units such as Cloud. Depending on the application, the Cloud can be in form of public, private, hybrid or community Cloud.

It will be obvious to those reasonably skilled in the art that other components performing the same functions may be suitably substituted. It should be mentioned that features explained with reference to a specific figure may be combined with features of other figures, even in those cases in which this has not explicitly been mentioned.

Spatially relative terms such as "under", "below", "lower", "over", "upper" and the like are used for ease of description to explain the positioning of one element relative to a second element. These terms are intended to encompass different orientations of the device in addition to different orientations than those depicted in the figures. Further, terms such as "first", "second", and the like, are also used to describe various elements, regions, sections, etc. and are also not intended to be limiting. Like terms refer to like elements throughout the description. As used herein, the terms "having", "containing", "including", "comprising" and the like are open ended terms that indicate the presence of stated elements or features, but do not preclude additional elements or features. The articles "a", "an" and "the" are intended to include the plural as well as the singular, unless the context clearly indicates otherwise.

With the above range of variations and applications in mind, it should be understood that the present invention is not limited by the foregoing description, nor is it limited by the accompanying drawings. Instead, the present invention is limited only by the following claims.

**Reference numbers**

| | | | |
|---|---|---|---|
| 10 | Cement preheater | 200 | Sensor device |
| 11 | Cement cyclone | 300 | Blockage evaluation unit |
| 11a | Cyclone barrel | 301 | Kiln specifications |
| 11b | Cyclone exit | 302 | Fuel quality data storage |
| 11c | Cyclone exhaust | 303 | Raw meal quality data storage |
| 11d | Cyclone entry | 304 | Residual sulphur data storage |
| 12 | Precalciner | 305 | Output to plant |
| 20 | Rotary kiln | 306 | Output to maintenance system |
| 31 | Kiln entrance | 310 | Hotmeal sulphur evaluation unit |
| 30 | Clinker cooler | 320 | Hotmeal alkali evaluation unit |
| 31 | Tertiary air pipe | 330 | Model selection unit |
| 32 | Air blower | 340 | Processing unit |
| 100 | Cement kiln | 370 | Action unit |
| $A_{hotmeal}$ | Hotmeal alkali content | | |
| B | Blockage | | |
| C | Clinker | | |
| $C_{fuel}$ | Fuel rate of consumption | | |
| $C_{rawmeal}$ | Raw meal rate of consumption | | |
| $C_{out}$ | Clinker rate of production | | |
| $E_{cooler}$ | Cooler exhaust | | |
| $E_{in}$ | Exhaust input | | |
| $E_{kiln}$ | Kiln exhaust | | |
| $E_{out}$ | Exhaust out | | |
| $F_{kiln}$ | Kiln fuel | | |
| $F_{calc}$ | Precalciner fuel | | |
| $H_{in}$ | Hotmeal input | | |
| $H_{out}$ | Hotmeal output | | |
| $P_{process}$ | Process pressure | | |
| $r_0$ | Radius of cyclone exit | | |
| $r_b$ | Radius of blockage | | |
| $R_{in}$ | Raw meal input | | |
| SAR | Sulphur-Alkali Ratio | | |
| $S_{clinker,\%}$ | Clinker sulphur content | | |
| $S_{fuel,\%}$ | Fuel sulphur content | | |
| $S_{hotmeal,\%}$ | Hotmeal sulphur content | | |
| $S_{rawmeal,\%}$ | Raw meal sulphur content | | |
| $S_{residual}$ | Residual sulphur | | |
| $T_{process}$ | Process temperature | | |
| $X_{alkali}$ | Hotmeal alkali quality values | | |
| $X_{fuel}$ | Fuel quality data values | | |

**Claims**

1.  A method for evaluating residual sulphur ($S_{residual}$) in a cement preheater (10) of a cement kiln (100), the method **characterised in that** it comprises:

    determining values of fuel sulphur content ($S_{fuel,\%}$) and fuel rate of consumption ($C_{fuel}$) of a fuel being provided to the cement kiln (100);
    determining a value of hotmeal quality ($Q_{hotmeal,\%}$) of a hotmeal being provided to the cement kiln;
    determining a value of clinker sulphur content ($S_{clinker,\%}$) of a clinker (C) being produced by the cement kiln (100); and

evaluating the residual sulphur ($S_{residual}$) in the cement preheater (10) using a sulphur evaluation unit (200), wherein the residual sulphur ($S_{residual}$) is based on the values of the fuel sulphur content ($S_{fuel,\%}$), the fuel rate of consumption ($C_{fuel}$), the hotmeal quality ($Q_{hotmeal,\%}$) and the clinker sulphur content ($S_{clinker,\%}$).

2. The method according to claim 1, wherein the hotmeal quality ($Q_{hotmeal,\%}$) comprises at least one of:

a hotmeal sulphur content ($S_{hotmeal,\%}$); and
a hotmeal alkali content ($A_{hotmeal,\%}$).

3. The method according to any one of claims 1 to 2, wherein the evaluating of the residual suphur ($S_{residual}$) is further based on at least one of:

a sulphur-alkali ratio (SAR) in the cement preheater (10);
a raw meal sulphur content ($S_{rawmeal,\%}$);
a raw meal alkali content ($A_{rawmeal,\%}$);
at least one temperature ($T_{process}$) at a predetermined location in the cement preheater (10); and
at least one pressure ($P_{process}$) at a predetermined location in the cement preheater (10),
and optionally wherein at least one of the following values is measured periodically in time:

the fuel sulphur content ($S_{fuel,\%}$);
the hotmeal quality ($Q_{hotmeal,\%}$); and
the clinker sulphur content ($S_{clinker,\%}$),
and further optionally wherein at least one of the respective periodically-measured values are upsampled, particularly temporally upsampled.

4. The method according to any one of claims 1 to 3, wherein the sulphur evaluation unit (200) is configured to evaluate the residual sulphur ($S_{residual}$) by a parameterized residual sulphur function ($f_1$, $f_2$, $f_3$) having adjustable model parameters, wherein the method preferably further includes adjusting the model parameters of the parameterized residual sulphur function.

5. The method according to any one of claims 1 to 4, wherein the cement preheater (10) comprises at least one cement cyclone (11) and optionally at least one cement precalciner (12).

6. A method for evaluating blockage (B) in a cement preheater (10), the method comprising:

evaluating the residual sulphur ($S_{residual}$) in the cement preheater (10) using the method according to any one of claims 1 to 5;
determining an agglomeration rate of sulphur compounds agglomerating on an inner surface of the cement preheater (10) based on the residual sulphur ($S_{residual}$); and
evaluating a level of blockage (B) in at least one predetermined pathway of the cement preheater (10) using a blockage evaluation unit (300), wherein the level of blockage (B) is based on the agglomeration rate,
and optionally wherein the blockage evaluation unit (300) is further configured to implement a gradient model for determining the agglomeration rate.

7. The method according to claim 6, wherein the blockage evaluation unit (300) is further configured for at least one of the determining the agglomeration rate and the evaluating the level of blockage (B) by a parameterized level of blockage function having adjustable model parameters, wherein the method preferably further includes adjusting the model parameters.

8. The method of claim 7, wherein the adjustable model parameters of the parameterized level of blockage function are adjusted based on training data comprising at least one of:

a maintenance-to-maintenance measurement wherein the rate of agglomeration is based on a first level of blockage measured at a first maintenance interval and a second level of blockage measured at a second maintenance interval; or
a maintenance-to-blockage measurement wherein the rate of agglomeration is based on a first level of blockage measured at a first maintenance interval and a level of complete blockage at a second interval.

9. The method of claim 7, wherein the adjustable model parameters of the parameterized level of blockage function are adjusted based on training data comprising at least one computational fluid dynamics simulation of the cement preheater (10), preferably wherein the computational fluid dynamics simulation is iterative.

10. The method of any one of claims 6 to 9, further comprising generating a signal indicating whether at least one of the residual sulphur ($S_{sulphur}$) and the level of blockage (B) has exceeded a threshold value, and optionally generating a command signal to initiate at least one of the following:

   scheduling or initiating a planned cleaning event, wherein the blockage is mechanically cleared, particularly by an air blast; and
   scheduling or initiating an SAR correction event, wherein the sulphur-to-alkali ratio (SAR) is corrected by introducing alkali into the cement preheater (10).

11. A sensor device for evaluating residual sulphur ($S_{residual}$) in a cement preheater (10) of a cement kiln (100), the sensor device **characterised in that** it comprises:

   a determining unit configured for determining values of fuel sulphur content ($S_{fuel,\%}$), fuel rate of consumption ($C_{fuel}$), hotmeal quality ($Q_{hotmeal,\%}$) and clinker sulphur content ($S_{clinker,\%}$); and
   a sulphur evaluation unit configured for evaluating the residual sulphur ($S_{residual}$) according to the method of any one of claims 1 to 5.

12. The sensor device according to claim 11, wherein the determining unit is configured for at least one of the following:

   receiving at least one of the values from a manual operator;
   receiving at least one of the values from a data store;
   measuring at least one of the values by receiving a signal from a physical sensor; and
   upsampling at least one of the values, particularly temporally upsampling at least one of the values.

13. The sensor device according to any one of claims 11 to 12, further comprising a network interface for connecting the sensor device to a data network, wherein the sensor device is operatively connected to the network interface for at least one of receiving at least one of the values, carrying out a command received from the data network, and sending status information of the sensor device to the data network.

14. A cement preheater (10) comprising:

   at least one cement cyclone (11);
   a sensor device for estimating residual Sulphur ($S_{residual}$) in the cement preheater (10) according to any one of claims 11 to 13; and
   a controller configured for carrying out the method for evaluating blockage (B) in the cement preheater (10) according to any one of claims 6 to 10.

15. A cement kiln (100) comprising the cement preheater (10) according to claim 14.


**Patentansprüche**

1. Verfahren zum Bewerten des Restschwefels ($S_{Rest}$) in einem Zementvorwärmer (10) eines Zementofens (100), wobei das Verfahren dadurch charakterisiert ist, dass es umfasst:

   Bestimmen von Werten des Brennstoffschwefelgehalts ($S_{Brennstoff,\%}$) und der Brennstoffverbrauchsrate ($C_{Brennstoff}$) eines Brennstoffs, der dem Zementofen bereitgestellt wird (100);
   Bestimmen eines Wertes der Heißmehlqualität ($Q_{Heißmehl,\%}$) eines Heißmehls, das dem Zementofen bereitgestellt wird;
   Bestimmen eines Wertes des Klinkerschwefelgehalts ($S_{Klinker,\%}$) eines Klinkers (C), der von dem Zementofen (100) produziert wird; und
   Bewerten des Restschwefels ($S_{Rest}$) in dem Zementvorwärmer (10) unter Verwendung einer Schwefelbewertungseinheit (200), wobei der Restschwefel ($S_{Rest}$) auf den Werten des Brennstoffschwefelgehalts ($S_{Brennstoff,\%}$), der Brennstoffverbrauchsrate ($C_{Brennstoff}$), der Heißmehlqualität ($Q_{Heißmehl,\%}$) und des Klinkerschwefelgehalts

$(S_{Klinker, \%})$ basiert.

2. Verfahren nach Anspruch 1, wobei die Heißmehlqualität $(Q_{Heißmehl, \%})$ mindestens eines der Folgenden umfasst:

Heißmehlschwefelgehalt $(S_{Heißmehl, \%})$; und
Heißmehlalkaligehalt $(A_{Heißmehl, \%})$.

3. Verfahren nach einem der Ansprüche 1 bis 2, wobei das Bewerten des Restschwefels $(S_{Rest})$ ferner auf mindestens einem der Folgenden basiert:

einem Schwefel-Alkali-Verhältnis (SAR) in dem Zementvorwärmer (10);
einem Schwefelgehalt im Rohmehl $(S_{Rohmehl, \%})$;
einem Alkaligehalt im Rohmehl $(A_{Rohmehl, \%})$;
mindestens einer Temperatur $(T_{Prozess})$ an einer vorbestimmten Stelle in dem Zementvorwärmer (10); und
mindestens einem Druck $(P_{Prozess})$ an einer vorbestimmten Stelle in dem Zementvorwärmer (10),
und wobei gegebenenfalls mindestens einer der folgenden Werte periodisch zeitgerecht gemessen wird:

der Brennstoffschwefelgehalt $(S_{Brennstoff, \%})$;
die Heißmehlqualität $(Q_{Heißmehl, \%})$; und
der Klinkerschwefelgehalt $(S_{Klinker, \%})$,
und wobei ferner gegebenenfalls mindestens einer der jeweiligen periodisch gemessenen Werte Upsampling unterzogen wird, insbesondere zeitweiligem Upsampling.

4. Verfahren nach einem der Ansprüche 1 bis 3, wobei die Schwefelbewertungseinheit (200) ausgelegt ist zum Bewerten des Restschwefels $(S_{Rest})$ durch eine parametrisierte Restschwefelfunktion $(f_1, f_2, f_3)$ mit justierbaren Modellparametern, wobei das Verfahren vorzugsweise weiterhin Justieren der Modellparameter der parametrierten Restschwefelfunktion umfasst.

5. Verfahren nach einem der Ansprüche 1 bis 4, wobei der Zementvorwärmer (10) mindestens einen Zementzyklon (11) und gegebenenfalls mindestens einen Zementvorkalzinator (12) umfasst.

6. Verfahren zum Bewerten von Blockierung (B) in einem Zementvorwärmer (10), wobei das Verfahren umfasst:

Bewerten des Restschwefels $(S_{Rest})$ in dem Zementvorwärmer (10) unter Verwendung des Verfahrens nach einem der Ansprüche 1 bis 5;
Bestimmen einer Agglomerationsrate von Schwefelverbindungen, die auf einer Innenoberfläche des Zementvorwärmers (10) agglomerieren, basierend auf dem Restschwefel $(S_{Rest})$; und
Bewerten eines Blockierungsgrads (B) in mindestens einem vorbestimmten Weg des Zementvorwärmers (10) unter Verwendung einer Blockierungsbewertungseinheit (300), wobei der Blockierungsgrad (B) auf der Agglomerationsrate basiert,
und wobei gegebenenfalls die Blockierungsbewertungseinheit (300) weiterhin ausgelegt ist zum Implementieren eines Gradientenmodells zum Bestimmen der Agglomerationsrate.

7. Verfahren nach Anspruch 6, wobei die Blockierungsbewertungseinheit (300) weiterhin ausgelegt ist für mindestens eines von dem Bestimmen der Agglomerationsrate und dem Bewerten des Blockierungsgrads (B) durch eine parametrierte Blockierungsgradfunktion mit justierbaren Modellparametern, wobei das Verfahren vorzugsweise weiterhin Justieren der Modellparameter einschließt.

8. Verfahren nach Anspruch 7, wobei die justierbaren Modellparameter der parametrierten Blockierungsgradfunktion basierend auf Trainingsdaten justiert werden, die mindestens eines der Folgenden umfassen:

eine Messung von Wartung bis Wartung, bei der die Agglomerationsrate auf einem ersten Blockierungsgrad, der bei einem ersten Wartungsintervall gemessen wird, und einem zweiten Blockierungsgrad basiert, der bei einem zweiten Wartungsintervall gemessen wird; oder
eine Messung von Wartung bis Blockierung, wobei die Agglomerationsrate auf einem ersten Blockierungsgrad, der bei einem ersten Wartungsintervall gemessen wird, und einem vollständigen Blockierungsgrad bei einem zweiten Intervall basiert.

9.  Verfahren nach Anspruch 7, wobei die justierbaren Modellparameter der parametrierten Blockierungsgradfunktion basierend auf Trainingsdaten justiert werden, die mindestens eine computergestützte Strömungsdynamiksimulation des Zementvorwärmers (10) umfassen, wobei vorzugsweise die computergestützte Strömungsdynamiksimulation iterativ ist.

10. Verfahren nach einem der Ansprüche 6 bis 9, weiterhin umfassend Generieren eines Signals, das angibt, ob mindestens eines von dem Restschwefel ($S_{Schwefel}$) und dem Blockierungsgrad (B) einen Schwellenwert überschritten hat,
    und gegebenenfalls Generieren eines Befehlssignals zum Initiieren von mindestens einem der Folgenden:

    Terminieren oder Initiieren eines geplanten Reinigungsereignisses, bei dem die Blockierung mechanisch beseitigt wird, insbesondere durch einen Luftstoß; und
    Terminieren oder Initiieren eines SAR-Korrekturereignisses, bei dem das Schwefel-Alkali-Verhältnis (SAR) durch Einbringen von Alkali in den Zementvorwärmer (10) korrigiert wird.

11. Sensorvorrichtung zum Bewerten des Restschwefels ($S_{Rest}$) in einem Zementvorwärmer (10) eines Zementofens (100), wobei die Sensorvorrichtung dadurch charakterisiert ist, dass sie Folgendes umfasst:

    eine Bestimmungseinheit, die ausgelegt ist zum Bestimmen der Werte des Brennstoffschwefelgehalts ($S_{Brennstoff, \%}$), der Brennstoffverbrauchsrate ($C_{Brennstoff}$), der Heißmehlqualität ($Q_{Heißmehl, \%}$) und des Klinkerschwefelgehalts ($S_{Klinker, \%}$); und
    eine Schwefelbewertungseinheit, die ausgelegt ist zum Bewerten des Restschwefels ($S_{Rest}$) nach einem der Ansprüche 1 bis 5.

12. Sensorvorrichtung nach Anspruch 11, wobei die Bestimmungseinheit ausgelegt ist für mindestens eines der Folgenden:

    Empfangen von mindestens einem Wert von einem manuellen Bediener;
    Empfangen von mindestens einem der Werte aus einem Datenspeicher;
    Messen von mindestens einem der Werte durch Empfangen eines Signals von einem physischen Sensor; und
    Upsampling von mindestens einem der Werte, insbesondere zeitweiliges Upsampling von mindestens einem der Werte.

13. Sensorvorrichtung nach einem der Ansprüche 11 bis 12, weiterhin umfassend eine Netzwerkschnittstelle zum Verbinden der Sensorvorrichtung mit einem Datennetzwerk, wobei die Sensorvorrichtung operativ für mindestens eines von Empfangen von mindestens einem der Werte, Durchführen eines Befehls, der von dem Datennetzwerk empfangen wird, und Senden von Statusinformationen der Sensorvorrichtung an das Datennetzwerk mit der Netzwerkschnittstelle verbunden ist.

14. Zementvorwärmer (10), umfassend:

    mindestens einen Zementzyklon (11);
    eine Sensorvorrichtung zum Schätzen von Restschwefel ($S_{Rest}$) in dem Zementvorwärmer (10) nach einem der Ansprüche 11 bis 13; und
    eine Steuerung, die ausgestaltet ist zum Durchführen des Verfahrens zum Bewerten von Blockierung (B) in dem Zementvorwärmer (10) nach einem der Ansprüche 6 bis 10.

15. Zementofen (100), umfassend den Zementvorwärmer (10) nach Anspruch 14.

**Revendications**

1.  Procédé d'évaluation du soufre résiduel ($S_{residual}$) dans un préchauffeur de ciment (10) d'un four à ciment (100), le procédé étant **caractérisé en ce qu'**il comprend :

    la détermination de valeurs de teneur en soufre de combustible ($S_{fuel, \%}$) et de taux de consommation de combustible ($C_{fuel}$) d'un combustible fourni au four à ciment (100) ;
    la détermination d'une valeur de qualité de farine chaude ($Q_{hotmeal, \%}$) d'une farine chaude fournie au four à ciment ;

la détermination d'une valeur de teneur en soufre de clinker ($S_{clinker,\%}$) d'un clinker (C) produit par le four à ciment (100) ; et

l'évaluation du soufre résiduel ($S_{residual}$) dans le préchauffeur de ciment (10) à l'aide d'une unité d'évaluation de soufre (200), le soufre résiduel ($S_{residual}$) étant basé sur les valeurs de la teneur en soufre de combustible ($S_{fuel,\%}$), le taux de consommation de combustible ($C_{fuel}$), la qualité de farine chaude ($Q_{hotmeal,\%}$) et la teneur en soufre de clinker ($S_{clinker,\%}$).

2. Procédé selon la revendication 1, dans lequel la qualité de farine chaude ($Q_{hotmeal,\%}$) comprend au moins un élément parmi :

une teneur en soufre de farine chaude ($S_{hotmeal,\%}$) ; et
une teneur en alcali de farine chaude ($A_{hotmeal,\%}$).

3. Procédé selon l'une quelconque des revendications 1 à 2, dans lequel l'évaluation du soufre résiduel ($S_{residual}$) est en outre basée sur au moins un élément parmi :

un rapport soufre-alcali (SAR) dans le préchauffeur de ciment (10) ;
une teneur en soufre de farine crue ($S_{rawmeal,\%}$) ;
une teneur en alcali de farine crue ($A_{rawmeal,\%}$) ;
au moins une température ($T_{process}$) au niveau d'un emplacement prédéterminé dans le préchauffeur de ciment (10) ; et
au moins une pression ($P_{process}$) au niveau d'un emplacement prédéterminé dans le préchauffeur de ciment (10), et facultativement dans lequel au moins une des valeurs suivantes est mesurée périodiquement dans le temps :

la teneur en soufre de combustible ($S_{fuel,\%}$) ;
la qualité de farine chaude ($Q_{hotmeal,\%}$) ; et
la teneur en soufre de clinker ($S_{clinker,\%}$),
et en outre facultativement dans lequel au moins une des valeurs mesurées périodiquement respectives est suréchantillonnée, en particulier suréchantillonnée temporellement.

4. Procédé selon l'une quelconque des revendications 1 à 3, dans lequel l'unité d'évaluation de soufre (200) est configurée pour évaluer le soufre résiduel ($S_{residual}$) par une fonction de soufre résiduel paramétrée ($f_1$, $f_2$, $f_3$) ayant des paramètres de modèle ajustables, le procédé incluant en outre préférablement l'ajustement des paramètres de modèle de la fonction de soufre résiduel paramétrée.

5. Procédé selon l'une quelconque des revendications 1 à 4, dans lequel le préchauffeur de ciment (10) comprend au moins un cyclone de ciment (11) et facultativement au moins un précalcinateur de ciment (12).

6. Procédé d'évaluation de blocage (B) dans un préchauffeur de ciment (10), le procédé comprenant :

l'évaluation du soufre résiduel ($S_{residual}$l) dans le préchauffeur de ciment (10) à l'aide du procédé selon l'une quelconque des revendications 1 à 5 ;
la détermination d'un taux d'agglomération de composés de soufre s'agglomérant sur une surface interne du préchauffeur de ciment (10) sur la base du soufre résiduel ($S_{residual}$) ; et
l'évaluation d'un niveau de blocage (B) dans au moins un passage prédéterminé du préchauffeur de ciment (10) à l'aide d'une unité d'évaluation de blocage (300), le niveau de blocage (B) étant basé sur le taux d'agglomération, et facultativement dans lequel l'unité d'évaluation de blocage (300) est en outre configurée pour mettre en œuvre un modèle de gradient afin de déterminer le taux d'agglomération.

7. Procédé selon la revendication 6, dans lequel l'unité d'évaluation de blocage (300) est en outre configurée pour au moins un élément parmi la détermination du taux d'agglomération et l'évaluation du niveau de blocage (B) par un niveau paramétré de fonction de blocage ayant des paramètres de modèle ajustables, le procédé incluant en outre préférablement l'ajustement des paramètres de modèle.

8. Procédé selon la revendication 7, dans lequel les paramètres de modèle ajustables du niveau paramétré de fonction de blocage sont ajustés sur la base de données d'apprentissage comprenant au moins un élément parmi :

une mesure de maintenance à maintenance dans lequel le taux d'agglomération est basé sur un premier niveau de blocage mesuré à un premier intervalle de maintenance et un second niveau de blocage mesuré à un second intervalle de maintenance ; ou

une mesure de maintenance à blocage dans lequel le taux d'agglomération est basé sur un premier niveau de blocage mesuré à un premier intervalle de maintenance et un niveau de blocage complet à un second intervalle.

9. Procédé selon la revendication 7, dans lequel les paramètres de modèle ajustables du niveau paramétré de fonction de blocage sont ajustés sur la base de données d'apprentissage comprenant au moins un élément parmi une simulation de mécanique des fluides numérique du préchauffeur de ciment (10), préférablement dans lequel la simulation de mécanique des fluides numérique est itérative.

10. Procédé selon l'une quelconque des revendications 6 à 9, comprenant en outre la génération d'un signal indiquant si au moins un élément parmi le soufre résiduel ($S_{sulphur}$) et le niveau de blocage (B) a dépassé une valeur seuil, et facultativement la génération d'un signal de commande pour déclencher au moins une des actions suivantes :

la programmation ou le déclenchement d'un événement de nettoyage planifié, dans lequel le blocage est dégagé mécaniquement, en particulier par un souffle d'air ; et

la programmation ou le déclenchement d'un événement de correction SAR, le rapport soufre-alcali (SAR) étant corrigé en introduisant de l'alcali dans le préchauffeur de ciment (10).

11. Dispositif capteur d'évaluation du soufre résiduel ($S_{residual}$) dans un préchauffeur de ciment (10) d'un four à ciment (100), le dispositif capteur étant **caractérisé en ce qu'**il comprend :

une unité de détermination configurée pour déterminer des valeurs de teneur en soufre de combustible ($S_{fuel,\%}$), un taux de consommation de combustible ($C_{fuel}$), une qualité de farine chaude ($Q_{hotmeal,\%}$) et une teneur en soufre de clinker ($S_{clinker,\%}$) ; et

une unité d'évaluation de soufre configurée pour évaluer le soufre résiduel ($S_{residual}$) selon le procédé de l'une quelconque des revendications 1 à 5.

12. Dispositif de capteur selon la revendication 11, dans lequel l'unité de détermination est configurée pour au moins une des actions suivantes :

la réception d'au moins une des valeurs en provenance d'un opérateur manuel ;

la réception d'au moins une des valeurs en provenance d'un magasin de données ;

la mesure d'au moins une des valeurs en recevant un signal en provenance d'un capteur physique ; et

le suréchantillonnage d'au moins une des valeurs, en particulier le suréchantillonnage temporel d'au moins une des valeurs.

13. Dispositif capteur selon l'une quelconque des revendications 11 à 12, comprenant en outre une interface réseau pour connecter le dispositif capteur à un réseau de données, le dispositif capteur étant connecté fonctionnellement à l'interface réseau pour au moins une action parmi la réception d'au moins une des valeurs, la réalisation d'une commande reçue en provenance du réseau de données, et l'envoi l'informations de statut du dispositif capteur au réseau de données.

14. Préchauffeur de ciment (10) comprenant :

au moins un cyclone de ciment (11) ;

un dispositif capteur pour estimer le soufre résiduel ($S_{residual}$) dans le préchauffeur de ciment (10) selon l'une quelconque des revendications 11 à 13 ; et

un dispositif de commande configuré pour réaliser le procédé d'évaluation de blocage (B) dans le préchauffeur de ciment (10) selon l'une quelconque des revendications 6 à 10.

15. Four à ciment (100) comprenant le préchauffeur de ciment (10) selon la revendication 14.

EP 4 449 039 B1

Fig. 1

Fig. 2

Fig. 3a

11a — — 11b

$r_b \approx r_0$, B = 0%

Fig. 3b

11a — — 11b

$r_b \approx 0.5 r_0$, B = 50%

Fig. 3c

11a — — 11b

$r_b \approx 0$, B = 100%

Fig. 4

— 200

$S_{hotmeal,\%}$

$C_{fuel}$, $S_{fuel,\%}$

$T_{process}$, $P_{process}$

$f_1$

$S_{hotmeal,\%}$

$f_3$

$S_{residual}$

SAR

$C_{rawmeal}$, $S_{rawmeal,\%}$
$T_{process}$, $P_{process}$

$f_2$

$S_{clinker,\%}$

$S_{clinker,\%}$

Fig. 5

**REFERENCES CITED IN THE DESCRIPTION**

**Patent documents cited in the description**

- JP H11262692 A
- WO 2013093245 A1
- WO 2017005697 A1
- WO 2019000807 A1